# EUROPEAN PATENT APPLICATION

(11) **EP 3 525 122 A1**
(43) Date of publication of application: **14.08.2019**
(21) Application number: 18156578.9
(22) Date of filing: 13.02.2018
(51) Int. Cl.: G06F 21/32, H04L 9/32, H04L 29/06

(54) **USING ULTRASOUND FOR IDENTIFICATION**

(71) Applicant: Koninklijke Philips N.V., 5656 AE Eindhoven (NL)
(72) Inventor: MAVROEIDIS, Dimitrios, 5656 AE Eindhoven (NL); GEBRE, Binyam Gebrekidan, 5656 AE Eindhoven (NL); VAN HEESCH, Christianus Martinus, 5656 AE Eindhoven (NL); COPPOLA, Giuseppe, 5656 AE Eindhoven (NL); DONATO, Katia, 5656 AE Eindhoven (NL)
(74) Representative: de Haan, Poul Erik

(57) **Abstract**

An apparatus (100) for identifying a subject is disclosed. The apparatus comprises an ultrasound sensor (102) for acquiring ultrasound data associated with a subject's wrist, a communications unit (104) for communicating with a database that stores validated ultrasound data associated with the wrists of one or more authenticated subjects, and a feature extraction engine (106) for determining whether the acquired ultrasound data matches the validated ultrasound data. A wearable device, a method and a computer program product are also disclosed.

## Description

### FIELD OF THE INVENTION

The invention relates to the identification of a subject and, more particularly to the identification of a subject using ultrasound.

### BACKGROUND OF THE INVENTION

It is often desirable to be able to identify a subject, for example to confirm that a particular subject is validated to perform a particular action. For example, it might be desirable to identify a subject who wishes to use a particular device (e.g. a wearable device) to ensure that the subject is authorized to use the device.

Various identification techniques are known in the field of user identification. Fingerprint identification techniques are readily used to authenticate a user of an electronic device, such as a smart phone. Fingerprint identification is useful due to the uniqueness of each fingerprint; there is a very small (almost negligible) chance that a third party (i.e. a subject other than an authorized subject) will be wrongly authenticated due to them having a matching fingerprint. However, a downside of fingerprint identification is that a user is typically required to place his or her finger or thumb on a fingerprint reader in order to be identified and authenticated. A further downside to fingerprint identification is that fingerprints can be replicated and, as a result, there exists the risk of a third party becoming authorized to use a device, for example by using copy or replica of an authorized user's fingerprint.

Therefore, it would be desirable to identify a subject using alternative, more discreet, easier to use and/or more secure technique.

### SUMMARY OF THE INVENTION

According to embodiments disclosed herein, biometric identification of a subject may be performed by identifying features obtained or extracted from ultrasound data associated with the subject's wrist. The inventors have realized that ultrasound data includes features which are unique to the subject from which the ultrasound data is obtained. Furthermore, ultrasound data obtained from a subject (e.g. an ultrasound image obtained from a scan) is difficult to replicate as structures within a subject's wrist are not easily accessible to third parties.

According to a first aspect, an apparatus for identifying the subject comprises an apparatus for identifying a subject, the apparatus comprising an ultrasound sensor for acquiring ultrasound data associated with a subject's wrist; a communications unit for communicating with a database that stores validated ultrasound data associated with the wrists of one or more authenticated subjects; and a feature extraction engine for determining whether the acquired ultrasound data matches the validated ultrasound data.

In some embodiments, the ultrasound sensor may comprise at least one of: an ultrasound imaging module for acquiring an ultrasound image of a subject's wrist; and an ultrasound sensor array for acquiring ultrasound data from a plurality of locations.

The feature extraction engine may use a segmentation algorithm to identify a set of structures within the subject's wrist. The feature extraction engine may be configured to determine that the acquired ultrasound data matches the validated ultrasound data if the identified set of structures match data associated with a corresponding set of structures stored in the database to within a threshold level of similarity.

In some embodiments, the feature extraction engine may comprise a machine learning engine. The machine learning engine may, in some embodiments, be configured to learn a function that maps features in the acquired ultrasound data to a vector space, wherein the mapping function generates a vector representative of the similarity of features in the acquired ultrasound data with corresponding features in the validated ultrasound data.

According to a second aspect, a wearable device is configured to be worn on a wrist of a subject, the wearable device comprising an apparatus as disclosed herein.

According to a third aspect, a method for identifying a subject comprises acquiring ultrasound data associated with a subject's wrist; and using a feature extraction algorithm to determine whether the acquired ultrasound data matches validated ultrasound data stored in a database, the database storing validated ultrasound data associated with the wrists of one or more authenticated subjects.

In some embodiments, the method may further comprise identifying, in the ultrasound data, a set of structures within the subject's wrist. The acquired ultrasound data may be determined to match validated ultrasound data if the identified set of structures matches data associated with a corresponding set of structures stored in the database to within a threshold level of similarity. The set of structures within the subject's wrist may include a portion of at least one of: a radial artery, a palmaris longus muscle, a pronator quadratus muscle, a flexor carpi ulnaris muscle, a tendon of the flexor carpi ulnaris muscle, a flexor pollicis longus muscle, a flexor digitorum sublimis muscle, a median nerve, an ulna bone and a radius bone.

The method may, in some embodiments, further comprise mapping features in the acquired ultrasound data to an embedded space; generating a vector representative of the similarity of each feature in the acquired ultrasound data with a corresponding feature in the validated ultrasound data; wherein the acquired ultrasound data is determined to match the validated ultrasound data if the features in the acquired ultrasound data match the corresponding features in the validated ultrasound data to within a threshold similarity.

In some embodiments, the method may further comprise, when the feature extraction algorithm determines that the acquired ultrasound data does match the validated ultrasound data stored in the database, enabling a function of a device. When the feature extraction algorithm determines that the acquired ultrasound data does not match the validated ultrasound data stored in the database, the method may comprise disabling or restricting a function of a device.

The feature extraction algorithm may comprise a machine learning algorithm.

In some embodiments, the method may further comprise training the machine learning algorithm using one of: a Siamese network arrangement; and a network having a triplet input arrangement, in which input reference data is paired with matching data and non-matching data.

According to a fourth aspect, a computer program product comprises a non-transitory computer readable medium, the computer readable medium having computer readable code embodied therein, the computer readable code being configured such that, on execution by a suitable computer or processor, the computer or processor is caused to perform one or more of the methods disclosed herein.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified schematic of an example of an apparatus for identifying a subject according to some embodiments;
Fig. 2 is a simplified illustration of an example of the apparatus of Fig. 1 and a wrist of a subject.
Fig. 3 is a simplified schematic of an example of a wearable device according to some embodiments;
Fig. 4 is a flowchart of an example of a method for identifying a subject according to some embodiments;
Fig. 5 is a flowchart of a further example of a method for identifying a subject according to some embodiments;
Fig. 6 is a flowchart of a further example of a method for identifying a subject according to some embodiments;
Fig. 7 is a flowchart of a further example of a method for identifying a subject according to some embodiments;
Fig. 8 is a flowchart of a further example of a method for identifying a subject according to some embodiments; and
Fig. 9 is a simplified schematic of an example of a computer-readable medium and a processor.

### DETAILED DESCRIPTION OF EMBODIMENTS

Embodiments disclosed herein relate to identifying a subject using ultrasound data acquired or obtained in respect of subject's wrist. Feature analysis may then be performed on the ultrasound data in order to extract features which can be used to identify the subject, for example by comparing the ultrasound data of the subject with previously-acquired ultrasound data which may, for example, be stored in a database. The subject may be identified if the ultrasound data of the subject matches previously-acquired ultrasound data stored in the database. In this way, the stored ultrasound data may be considered to be validated data, as the subject or subjects from whom the stored data was obtained is known.

In the context of embodiments disclosed herein, "identifying a subject" is intended to refer to determining whether or not a subject has been validated; i.e. determining whether or not data associated with the subject corresponds to data of known subjects stored in the database. "Identifying a subject" does not necessarily require the identity (e.g. personal details such as name, date of birth, and so on) to be determined in respect of the subject.

Various feature extraction methods may be used for identifying the subject in the embodiments disclosed herein. Some embodiments use machine learning techniques in order to identify the subject. Various machine learning algorithms will be known to those skilled in the art. Examples of such techniques include artificial neural networks and deep neural networks. Artificial neural networks (also referred to simply as neural networks) will be familiar to those skilled in the art, but in brief, a neural network is a type of model that can be used to classify data (for example, classify, or identify the contents of data such as ultrasound data). The structure of a neural network is inspired by the human brain. Neural networks are comprised of layers, each layer comprising a plurality of neurons. Each neuron comprises a mathematical operation. In the process of classifying a piece of data, the mathematical operation of each neuron is performed on the data to produce a numerical output, and the outputs of each layer in the neural network are fed into the next layer sequentially. Generally, the mathematical operations associated with each neuron comprise one or more weights that are tuned during the training process (e.g. the values of the weights are updated during the training process to tune the model to produce more accurate classifications).

For example, in a neural network model for classifying the contents of images, each neuron in the neural network may comprise a mathematical operation comprising a weighted linear sum of the pixel (or in three dimensions, voxel) values in the image followed by a non-linear transformation. Examples of non-linear transformations used in neural networks include sigmoid functions, the hyperbolic tangent function and the rectified linear function. The neurons in each layer of the neural network generally comprise a different weighted combination of a single type of transformation (e.g. the same type of transformation, sigmoid etc. but with different weightings). As will be familiar to the skilled person, in some layers, the same weights may be applied by each neuron in the linear sum; this applies, for example, in the case of a convolution layer. The weights associated with each neuron may make certain features more prominent (or conversely less prominent) in the classification process than other features and thus adjusting the weights of neurons in the training process trains the neural network to place increased significance on specific features when classifying an image. Generally, neural networks may have weights associated with neurons and/or weights between neurons (e.g. that modify data values passing between neurons).

During training, the neural network processes examples of data that have been processed (e.g. annotated or classified) by a human annotator (training data). For each piece of training data, the neural network processes the training data, compares the classification produced by the neural network with the classification provided by the human annotator and updates the weights in order to improve the classification. Thus, in context of the embodiments disclosed herein, the training data may include ultrasound data (e.g. ultrasound scan images) acquired from scanning the wrists of various subjects, the various features and structures identifiable in the ultrasound data having been annotated or classified by a human annotator.

Examples of suitable neural networks include, but are not limited to feed forward models (such as convolutional neural networks, auto-encoder neural network models, probabilistic neural network models and time delay neural network models), radial basis function network models, recurrent neural network models (such as fully recurrent models, Hopfield models, or Boltzmann machine models), or any other type of neural network model comprising weights.

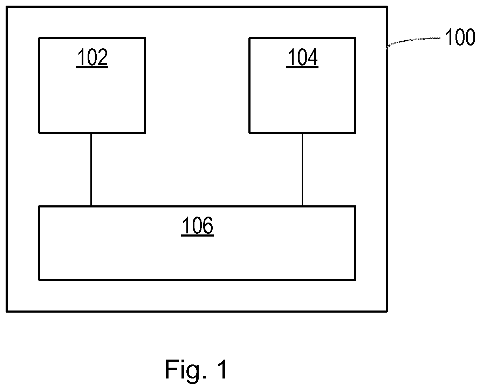

Various embodiments will now be described with reference to the figures. Fig. 1 shows, schematically, an apparatus 100 for identifying a subject. The apparatus 100 comprises an ultrasound sensor 102, a communications unit 104 and a feature extraction engine 106. The apparatus 100 may, in some embodiments, further comprise other components, such as a memory (not shown) and/or a processor or controller (not shown) for controlling one or more of the other components of the apparatus. In some embodiments, the memory may store computer readable code which can be executed by the processor to enable the apparatus 100 to function and perform the methods disclosed herein.

The ultrasound sensor 102 is for acquiring ultrasound data associated with a subject's wrist. For example, the ultrasound sensor 102 may comprise an ultrasound scanner capable of or configured to capture ultrasound data from the wrist of the subject. In some embodiments, the ultrasound sensor may comprise at least one of: an ultrasound imaging module for acquiring an ultrasound image of a subject's wrist; and an ultrasound sensor array for acquiring ultrasound data from a plurality of locations. An ultrasound imaging module may enable a thorough (e.g. high-detail or high-resolution) ultrasound image of the wrist to be obtained. However, such an imaging module may be relatively expensive, may consume large amounts of power and/or may be relatively large, therefore taking up valuable space in any device in which it is implemented. An ultrasound sensor array, on the other hand, may be less expensive and may take up less space in a device, but may capture ultrasound data at a relatively lower resolution. An ultrasound sensor array may include a plurality of individual ultrasound sensors (e.g. individual point sensors) arranged in an array. The data acquired by each individual sensor may be knitted together in order to obtain an ultrasound image of the wrist.

In some embodiments, the ultrasound sensor 102 may be configured such that ultrasound data associated with a subject's wrist can be acquired only when the ultrasound sensor is positioned adjacent to (e.g. in contact with) the subject's wrist.

The communications unit 104 may be capable of or configured to communicate with a database that stores validated ultrasound data associated with the wrists of one or more authenticated subjects. The communications unit 104 may, for example, comprise a transceiver associated with suitable processing circuitry to enable acquired ultrasound data to be compared with previously-acquired ultrasound data stored in the database. The communications unit 104 may enable wired or wireless communications between the apparatus 100 and the database. In some examples, a database may be located remotely with respect to the apparatus 100. For example, the database may be stored in a memory located in a remote computing device or on a server. The database may, in some embodiments, be stored in a cloud computing environment accessible by the communications unit 104. In other examples, the database may be located in the apparatus 100 itself. For example, the database may be stored in a memory unit (not shown) within the apparatus 100.

The feature extraction engine 106 may be capable of or configured to determine whether the acquired ultrasound data matches the validated ultrasound data. Various techniques may be used by the feature extraction engine 106 in order to make a determination. For example, one relatively straightforward implementation of the feature extraction engine 106 may involve comparing acquired ultrasound data (e.g. an ultrasound image) with an ultrasound image which has been acquired previously from the subject and stored in the database. If the acquired ultrasound image matches the previously-acquired ultrasound image, then the identity of the subject may be confirmed. In an alternative implementation, the feature extraction engine 106 may extract features from the acquired ultrasound data and compare those features with corresponding features in the previously-acquired ultrasound data in the database. If the compared features match, then the identity of the subject may be confirmed. Other implementations of the feature extraction engine 106 are discussed below.

In some embodiments, the feature extraction engine 106 may use a segmentation algorithm to identify a set of structures within the subject's wrist. For example, the feature extraction engine 106 may apply image segmentation methods to segment the acquired ultrasound data (e.g. an ultrasound image) for further analysis. Each segment of the image resulting from the image segmentation may include a structure in the set of structures. The set of structures may, for example, include anatomical structures within the wrist of the subject which can be used to identify the subject. Each structure may have a particular size and/or shape which may differ from subject to subject. Furthermore, the relative positions and/or orientations of a set of structures in the wrist of one subject may be unique with respect to other subjects' wrists. Consequently, by comparing the arrangement of a set of structures identified in the acquired ultrasound data with the arrangement of a corresponding set of structures in the previously-acquired ultrasound data, it is possible to determine whether or not a match exists. The intention is that one or more authorized/validated subjects will have ultrasound data associated with the wrists stored in the database. If the newly-acquired ultrasound data associated with one of those subjects is compared with data in the database, then a positive match should result, meaning that the subject can be validated and, for example, may be authorized to perform a particular task. However, if the newly-acquired ultrasound data is from a subject other than those whose data is stored in the database, then the subject from whom the newly-acquired data is obtained may not be validated and, for example, may not be authorized to perform a particular task.

Image segmentation techniques will be known to those skilled in the art. Briefly, however, image segmentation involves partitioning image data (e.g. the ultrasound data obtained from the rest of a subject) into multiple segments which can be used for further analysis. By segmenting the image into smaller portions, analysis of the whole image is a simplified as each smaller portion of the image can be analyzed separately. Thus, the ultrasound image data may be segmented into a plurality of segments, each segment including one or more structures in the subject's wrist.

In some embodiments, the feature extraction engine 106 may be configured to determine that the acquired ultrasound data matches the validated ultrasound data if the identified set of structures match data associated with a corresponding set of structures stored in the database to within a threshold level of similarity. In other words, it may not be necessary for every structure in the identified set of structures of the acquired image data to match exactly with the corresponding set of structures in the data stored in the database. Over time, structures (e.g. muscles, bones, tendons and the like) may change size and/or may move relative to one another, for example due to growth or injury. Therefore, minor changes in the arrangement of structures in the subject's wrist may be ignored or disregarded if, for example, a significant proportion of the structures do match. In some embodiments, a degree or level of similarity between the acquired ultrasound data and the previously-acquired validated ultrasound data (i.e. the validated data in the database) may be determined. In such cases, the feature extraction engine 106 may determine that the acquired data matches validated data in the database if the determined level of similarity exceeds the threshold level of similarity. The threshold level of similarity may be chosen based on the requirements of the system (e.g. based on how close a match is needed). In some examples, the threshold level of similarity may be 75%, 80%, 85%, 90% or 95%. In other examples, other threshold levels may be used.

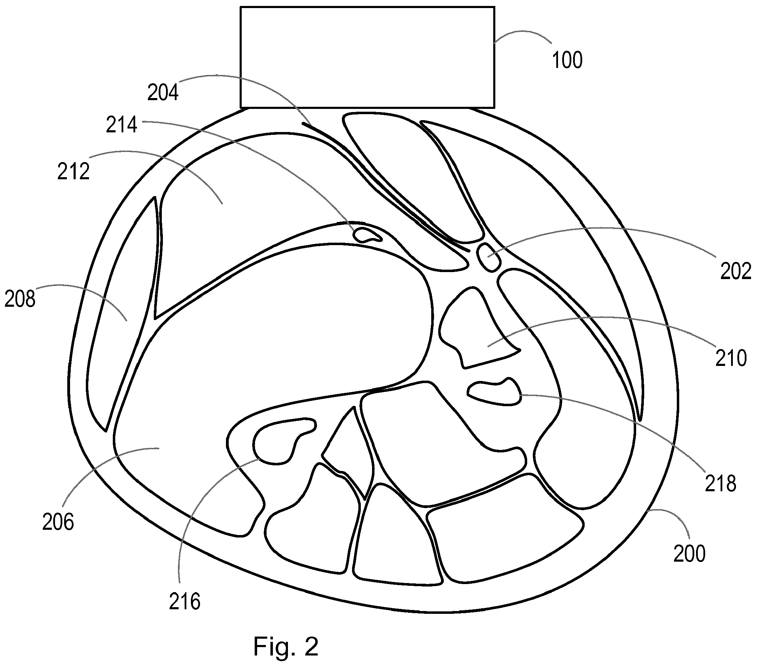

Fig. 2 shows, schematically, an example of the apparatus 100 mounted on a wrist 200 of a subject. The wrist 200 is shown in cross-section, and various structures within the subject's wrist are identified. The set of structures identified by the feature extraction engine 106 (e.g. using the segmentation algorithm) may include any structures of a wrist. In some embodiments, the set of structures may include a portion of at least one of a radial artery 202, a palmaris longus muscle 204, a pronator quadratus muscle 206, a flexor carpi ulnaris muscle 208, a tendon of the flexor carpi ulnaris muscle, a flexor pollicis longus muscle 210, a flexor digitorum sublimis muscle 212, a median nerve 214, an ulna bone 216 and a radius bone 218. Other structures of the wrist may be included in the set of structures in addition to, or instead of, those structures listed above.

Referring again to Fig. 1, the feature extraction engine 106 may, in some embodiments, comprise a machine learning engine. As discussed briefly above, machine learning techniques may use a neural network model, such as a deep neural network model. Such a neural network model may first be trained, as noted above, by inputting a set of training data which includes ultrasound data (e.g. ultrasound images) associated with wrists of various subjects. The data used to train the neural network model includes annotations or classifications which may, for example, have been added by a medical professional. The annotations or classifications may indicate that a particular image, or set of image data, belongs to a particular subject. The training data is used by the neural network model to learn which features within the data can be used to identify subjects from other ultrasound data in the future. The neural network model learns to ignore "noise" in the data, and focus on those features from which a positive identification (i.e. a data match) can be made. In this context, "noise" in the data may include all differences, such as slight changes in the shape, size and/or position of various features and/or structures identified in the data. Such minor changes may occur naturally over time, and the presence of such a different between acquired data and previously acquired data should not be to a negative identification result if the acquired data and the previously-acquired data is in fact associated with the same subject.

The machine learning engine (e.g. a neural network model) may be used to determine whether the acquired data matches validated data (i.e. previously-acquired data from the same subject) in a number of ways. According to some embodiments, the machine learning engine may implement image segmentation as discussed above. In some examples, a deep learning segmentation algorithm may be used. As noted in the examples described above, an image segmentation algorithm is applied to ultrasound data (e.g. ultrasound image data) in order to segment the data into smaller, easier to analyze portions. During a training phase, turning data may be segmented and provided as an input to a neural network model (e.g. a deep neural network model), so that the neural network model can learn how to classify (e.g. recognize) features in individual segments of the data. For example, the neural network model may learn to classify individual structures of the wrist which are identified in the ultrasound data.

During training of a neural network model, details of each identified structure (e.g. a classification of each structure) and spatial relationships between the identified structures (e.g. distances between structures and positions of structures relative to one another) may be learnt and stored for each subject. Thus, for each validated subject (i.e. each subject for whom it is intended has a matching data set in the database), a record of the identified structures and the details (e.g. classifications, and relative distances and positions) is stored in the database. In some embodiments, the data is stored for each subject in the form of a template, against which newly-acquired data may be compared for validation.

After the neural network model has been trained, an apparatus such as the apparatus 100 may use the trained neural network model to determine whether newly-acquired data matches with data stored in the database. In some embodiments, the neural network model may, in use (referred to as a deployment phase), apply a segmentation algorithm to the newly-acquired ultrasound data to identify the set of structures, then find the most similar stored data record (e.g. template) in the database to the acquired data. Since the neural network model has been trained, it is able to find the closest-matching data record or template, focusing on important features and aspects, and disregarding any minor differences, such as minor differences in size and/or position of structures which might be expected to change size or move over time. Thus, the neural network may reliably locate a matching data record in the database if one exists (i.e. if data for the subject has been previously stored in the database).

In some embodiments, the neural network model may determine that a data record in the database matches a newly-acquired data set if there exists a sufficient similarity between the data. In other words, the machine learning engine may be configured to determine that the acquired ultrasound data matches the validated ultrasound data if the features in the acquired ultrasound data match the corresponding features in the validated ultrasound data to within a threshold level of similarity. The threshold level may be chosen or defined according to a desired level of security or authentication. For example, a high threshold level of similarity may be set in cases where near certainty of a match is required in order to validate a subject. In cases where a lower level of certainty is required, a lower threshold level may be set.

In some embodiments the machine learning engine may, in addition to, or as an alternative to, the image segmentation algorithm, implement a similarity learning algorithm, which uses vector space mapping techniques. The similarity learning algorithm involves the mapping of features extracted from the acquired ultrasound data to a vector space (also referred to as an embedded space). In other words, features extracted from the ultrasound data, and relationships between the extracted features are represented as a plurality of vectors. The vector or embedded space to which the features are mapped enables similarities to be determined by comparing corresponding vectors with one another. The general goal is that the similarity between the vector mappings in the vector space/embedded space is appropriate for the biometric identification task; i.e. the ultrasound scans taken from one person are mapped to nearby points while scans taken from different people are mapped to faraway points.

A neural network model, such as a deep neural network may be implemented for the similarity learning technique. During training, a deep learning algorithm may learn and store the mapping of features in the ultrasound data to the vector space for each subject. Thus, for each validated subject (i.e. each subject for whom it is intended matching data set is stored in the database), a record of the feature-mapping to the vector space is stored in the database. Using the vector space mapping method, features from the same subject are matched to points in the vector space that are near to one another while features from difference subjects are mapped to points in the vector space that are further away from one another.

After training, an apparatus such as the apparatus 100 may use the trained neural network model to determine whether newly-acquired data matches with data stored in the database. In some embodiments, in use (i.e. during the deployment phase), the neural network model may map features extracted from newly-acquired data the vector space, then find the most similar stored data record in the database to the acquired data. The most similar stored data record may be located by comparing the vectors for each feature.

Again, in some embodiments, a threshold level of similarity may be applied, such that machine learning engine may be configured to determine that the acquired ultrasound data matches the validated ultrasound data if the features in the acquired ultrasound data match the corresponding features in the validated ultrasound data to within a threshold level of similarity.

Thus, according to some embodiments, the machine learning engine may be configured to learn a function that maps features in the acquired ultrasound data to a vector space, wherein the mapping function generates a vector representative of the similarity of features in the acquired ultrasound data with corresponding features in the validated ultrasound data.

In embodiments in which machine learning techniques are employed, various methods may be used to train the neural network or deep neural network. In some embodiments, a "Siamese neural network" arrangement may be implemented. A Siamese neural network includes two (or more) identical subnetworks, having the same configuration and the same parameters and weights. Each network processes one of the sets of data for which a similarity analysis is required (i.e. the acquired data and the validated data). The Siamese neural network generates the mapping of the features in the data to the vector space as described above. In other embodiments, a triplet input neural network arrangement may be implemented. In such an arrangement, a three sets of data are input to the neural network model in two pairs: a first pair includes an input reference data set (i.e. the acquired ultrasound data for a subject) and a matching data set (i.e. validated ultrasound data for the same subject), and a second pair includes the input reference data set and a non-matching data set (i.e. ultrasound data for a different subject). In this way, the neural network can learn which features in the data are similar between the data sets, and also which features in the data cause them the data sets not to match.

The apparatus 100 described above is configured to identify a subject based on ultrasound data associated with (e.g. acquired from) a subject's wrist. If the data matches with previously-acquired validated data in a database, the subject may be considered to be authenticated, or authorized to, for example, use the apparatus. In this way, the apparatus 100 may be referred to as an authentication apparatus.

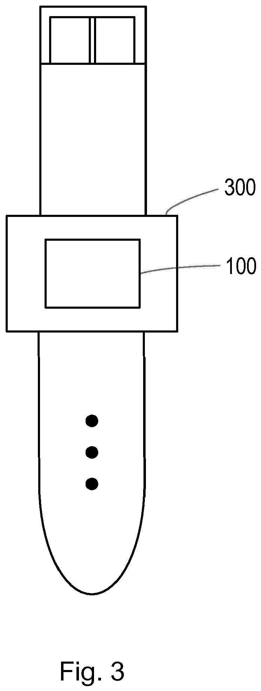

During use, the apparatus, or authentication apparatus 100, acquires ultrasound data associated with the subject's wrist, using the ultrasound sensor 102. Thus, the apparatus 100 may usefully be positioned near to the subject's wrist during use to aid the data acquisition from the wrist. In some embodiments, the apparatus may be implemented in, incorporated into, attached to or otherwise associated with, a wearable device. For example, the apparatus 100 may form part of a device configured to be worn on the wrist of a subject, so that an ultrasound scan can be performed easily. Thus, another aspect relates to such a wearable device. Fig. 3 is a simplified schematic of an example of a wearable device 300. The wearable device 300 may be a wearable authentication device. In some embodiments, the wearable device 300 may be configured to be worn on the wrist of a subject. The wearable device may, for example, comprise a watch, a fitness tracker, a wearable health device or tracker, a smart watch, or the like. The wearable device 300 comprises an apparatus for identifying a subject, such as the apparatus 100. Thus, a subject may wear the wearable device 300, and the apparatus 100 may capture ultrasound data from the subject's wrist, then perform a validation check to determine whether the subject's ultrasound data is stored in the database. If the subject's data does match data stored in the database, then the subject may, for example, be authorized to operate the wearable device 300. If no match for the subject's ultrasound data is found in the database, then it may be determined that the subject is not authorized and, as a result, the subject may be prevented from operating the wearable device. Such an arrangement may add a level of security to the wearable device, which may be particularly useful if the wearable device were to contain personal or confidential data regarding the subject, such as medical or health data.

In order to validate the subject, such that the subject is authorized to perform a particular action or use a particular device, ultrasound data associated with the subject's wrist may be acquired from the subject, for example during an initiation phase the first time the subject uses the apparatus 100 or the wearable device 300. The ultrasound data associated with the subject's wrist may then be stored in the database for subsequent validation use.

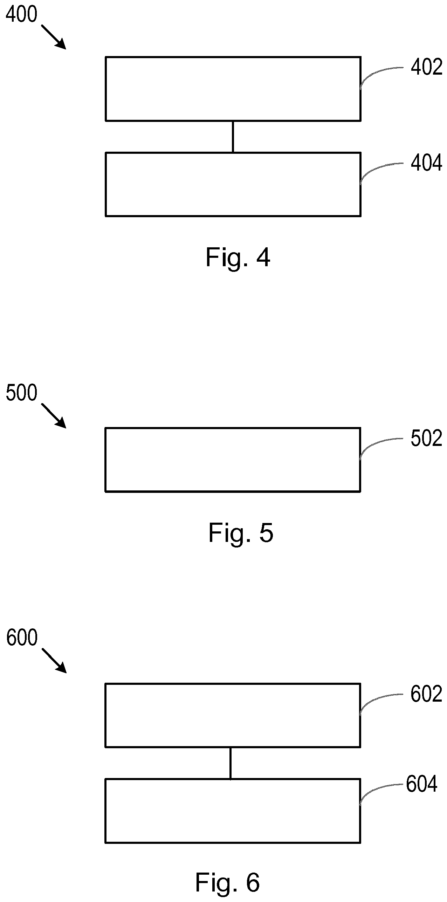

A further aspect relates to a method for identifying a subject. Fig. 4 is a flowchart of an example of a method 400 for identifying a subject. The method 400 comprises, at step 402, acquiring ultrasound data associated with a subject's wrist. At step 404, the method 400 comprises using a feature extraction algorithm to determine whether the acquired ultrasound data matches validated ultrasound data stored in a database, the database storing validated ultrasound data associated with the wrists of one or more authenticated subjects. The apparatus 100 may be arranged to perform the method 400.

In some embodiments, the ultrasound data associated with the subject's wrist may be acquired from or by a source other than the ultrasound sensor in the apparatus 100. For example, the data may be acquired by another scanner or device, and transmitted to the feature extraction engine via a network, such as the internet. Similarly, while, in some embodiments, the database may be located in a memory within the apparatus 100, in other embodiments, the database may be located remotely from the apparatus, such as in a remote server. The feature extraction engine may, in some embodiments, apply the feature extraction algorithm.

Fig. 5 is a flowchart of a further example of a method 500 for identifying a subject. The method 500 may include steps of the method 400. The method 500 may comprise, at step 502, identifying, in the ultrasound data, a set of structures within the subject's wrist. The acquired ultrasound data may be determined to match validated ultrasound data if the identified set of structures matches data associated with a corresponding set of structures stored in the database to within a threshold level of similarity. As noted above, the threshold may be defined to suit the authentication requirements or desires.

As with the examples described above, the set of structures may include any structures of the wrist, such as those shown in Fig. 2. Specifically, the set of structures within the subject's wrist 200 may include a portion of at least one of: a radial artery 202, a palmaris longus muscle 204, a pronator quadratus muscle 206, a flexor carpi ulnaris muscle 208, a tendon of the flexor carpi ulnaris muscle, a flexor pollicis longus muscle 210, a flexor digitorum sublimis muscle 212, a median nerve 214, an ulna bone 216 and a radius bone 218.

In addition to, or as an alternative to the step 502 discussed above, a feature mapping method may be implemented as discussed above. Fig. 6 is a flowchart of a further example of a method 600 for identifying a subject. The method 600 may include steps of the methods described above. The method 600 may comprise, at step 602, mapping features in the acquired ultrasound data to an embedded space (also referred to as a vector space or latent space). At step 604, the method may comprise generating a vector representative of the similarity of each feature in the acquired ultrasound data with a corresponding feature in the validated ultrasound data. The acquired ultrasound data may be determined to match the validated ultrasound data if the features in the acquired ultrasound data match the corresponding features in the validated ultrasound data to within a threshold similarity.

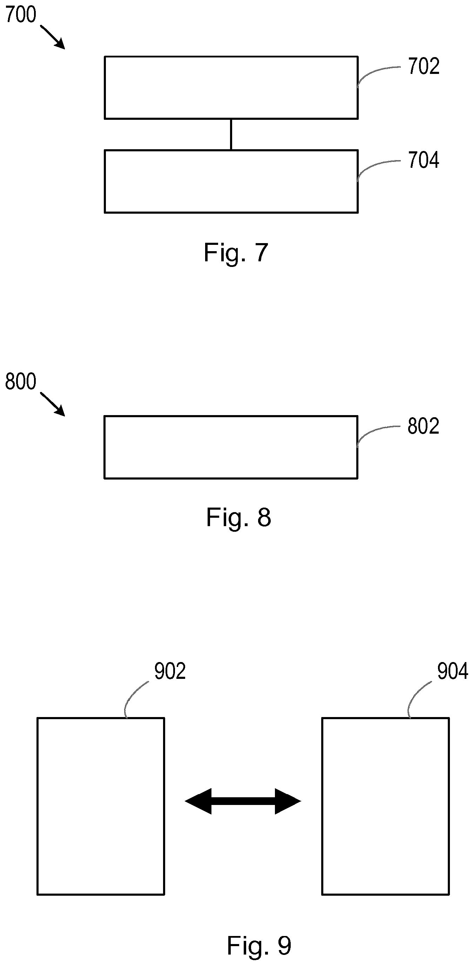

The apparatus 100 and the methods disclosed herein may be considered as authentication apparatus and authentication methods, since they can be used to authenticate or validate a subject from whom ultrasound data can be acquired. When the apparatus or methods are used in association with a device, they may be used to validate the subject as an authorized user of the device. Thus, in some embodiments, some functionality of a device may be enabled or disabled in respect of a subject depending on whether or not the subject is validated/authorized. Fig. 7 is a flowchart of a further example of a method 700 for identifying a subject. The method 700 may include steps of the methods described above. According to the method 700, when the feature extraction algorithm determines that the acquired ultrasound data does match the validated ultrasound data stored in the database, the method may comprise, at step 702, enabling a function of a device. Similarly, when the feature extraction algorithm determines that the acquired ultrasound data does not match the validated ultrasound data stored in the database, the method 700 may comprise, at step 702, disabling or restricting a function of a device. For example, the function of the device may include allowing access to personal data concerning the subject, or opening a user interface of the device.

As will be apparent from the above discussion of the apparatus 100, the feature extraction algorithm used in the methods described herein may comprise a machine learning algorithm. The machine learning algorithm may be configured to perform one or more of the various machine learning techniques discussed above. According to some embodiments, the machine learning algorithm may be trained using any of the training methods discussed above. Fig. 8 is a flowchart of a further example of a method 800 for identifying a subject. The method 800 may include steps of the methods discussed above. The method 800 may comprise, at step 802, training the machine learning algorithm using one of: a Siamese network arrangement; and a network having a triplet input arrangement, in which input reference data is paired with matching data and non-matching data. The Siamese network arrangement and the triplet input arrangement may be implemented according to the discussions above.

A further aspect relates to a computer program product. Fig. 9 is a simplified schematic of an example of a computer-readable medium 902 and a processor 904. The computer program product comprises a non-transitory computer readable medium 902, the computer readable medium having computer readable code embodied therein, the computer readable code being configured such that, on execution by a suitable computer or processor 904, the computer or processor is caused to perform one or more of the methods disclosed herein.

The processor 904 can comprise one or more processors, processing units, multi-core processors or modules that are configured or programmed to control the apparatus 100 in the manner described herein. In particular implementations, the processor 904 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for identifying a subject, the apparatus comprising:
an ultrasound sensor (102) for acquiring ultrasound data associated with a subject's wrist;
a communications unit (104) for communicating with a database that stores validated ultrasound data associated with the wrists of one or more authenticated subjects; and
a feature extraction engine (106) for determining whether the acquired ultrasound data matches the validated ultrasound data.

2. An apparatus (100) according to claim 1, wherein the ultrasound sensor (104) comprises at least one of: an ultrasound imaging module for acquiring an ultrasound image of a subject's wrist; and an ultrasound sensor array for acquiring ultrasound data from a plurality of locations.

3. An apparatus (100) according to claim 1 or claim 2, wherein the feature extraction engine (106) uses a segmentation algorithm to identify a set of structures within the subject's wrist.

4. An apparatus (100) according to claim 3, wherein the feature extraction engine (106) is configured to determine that the acquired ultrasound data matches the validated ultrasound data if the identified set of structures match data associated with a corresponding set of structures stored in the database to within a threshold level of similarity.

5. An apparatus (100) according to any of the preceding claims, wherein the feature extraction engine (106) comprises a machine learning engine.

6. An apparatus (100) according to claim 5, wherein the machine learning engine is configured to learn a function that maps features in the acquired ultrasound data to a vector space, wherein the mapping function generates a vector representative of the similarity of features in the acquired ultrasound data with corresponding features in the validated ultrasound data.

7. A wearable device (300) configured to be worn on a wrist of a subject, the wearable device comprising an apparatus (100) according to any of the preceding claims.

8. A method (400) for identifying a subject, comprising:
acquiring (402) ultrasound data associated with a subject's wrist; and
using (404) a feature extraction algorithm to determine whether the acquired ultrasound data matches validated ultrasound data stored in a database, the database storing validated ultrasound data associated with the wrists of one or more authenticated subjects.

9. A method (500) according to claim 8, further comprising:
identifying (502), in the ultrasound data, a set of structures within the subject's wrist;
wherein the acquired ultrasound data is determined to match validated ultrasound data if the identified set of structures matches data associated with a corresponding set of structures stored in the database to within a threshold level of similarity.

10. A method according to claim 9, wherein the set of structures within the subject's wrist (200) includes a portion of at least one of: a radial artery (202), a palmaris longus muscle (204), a pronator quadratus muscle (206), a flexor carpi ulnaris muscle (208), a tendon of the flexor carpi ulnaris muscle, a flexor pollicis longus muscle (210), a flexor digitorum sublimis muscle (212), a median nerve (214), an ulna bone (216) and a radius bone (218).

11. A method (600) according to any of claims 8 to 10, further comprising:
mapping (602) features in the acquired ultrasound data to an embedded space;
generating (604) a vector representative of the similarity of each feature in the acquired ultrasound data with a corresponding feature in the validated ultrasound data;
wherein the acquired ultrasound data is determined to match the validated ultrasound data if the features in the acquired ultrasound data match the corresponding features in the validated ultrasound data to within a threshold similarity.

12. A method (700) according to any of claims 8 to 11, further comprising:
when the feature extraction algorithm determines that the acquired ultrasound data does match the validated ultrasound data stored in the database:
enabling (702) a function of a device; and
when the feature extraction algorithm determines that the acquired ultrasound data does not match the validated ultrasound data stored in the database:
disabling (704) or restricting a function of a device.

13. A method according to any of claims 8 to 12, wherein the feature extraction algorithm comprises a machine learning algorithm.

14. A method (800) according to claim 13, further comprising:
training(802) the machine learning algorithm using one of: a Siamese network arrangement; and a network having a triplet input arrangement, in which input reference data is paired with matching data and non-matching data.

15. A computer program product comprising a non-transitory computer readable medium, the computer readable medium (902) having computer readable code embodied therein, the computer readable code being configured such that, on execution by a suitable computer or processor (904), the computer or processor is caused to perform the method of any claims 8 to 14.
